# EUROPEAN PATENT APPLICATION

(11) **EP 4 717 712 A2**
(43) Date of publication of application: **01.04.2026**
(21) Application number: 26155778.9
(22) Date of filing: 27.12.2022
(51) Int. Cl.: C07K 16/28

(54) **VEDOLIZUMAB FORMULATION**

(30) Priority: 27.12.2021 EP 21217815
(62) Divisional of application: 22844497.2
(71) Applicant: Polpharma Biologics S.A., 80-172 Gdansk (PL)
(72) Inventor: JAREMICZ, Zbigniew, 80-172 Gdansk (PL); SUCHY, Dariusz, 80-172 Gdansk (PL)
(74) Representative: Simmons & Simmons LLP (Munich)

(57) **Abstract**

The present invention is directed to pharmaceutical formulations comprising the anti-α4β7 integrin antibody Vedolizumab. The formulations comprise a specific concentration of sorbitol and shows improved stability properties. Furthermore, the present invention concerns products comprising said formulations and uses thereof for the treatment of various diseases.

## Description

### FIELD OF THE INVENTION

The present invention pertains to the field of antibody formulations, especially formulations comprising anti-α4β7 integrin antibodies, such as Vedolizumab. More specifically, the present invention is directed to pharmaceutical formulations comprising the antibody Vedolizumab suitable for subcutaneous administration, as well as related products and uses for the treatment of various diseases and disorders.

### BACKGROUND OF THE INVENTION

Therapeutic preparations comprising proteins, especially antibodies, as active ingredient have become more and more important over the past decades. Most of the therapeutic antibodies are administered via parenteral routes, such as by intravenous infusions or by subcutaneous or intramuscular injection. Stability of the antibody in the formulation is a major concern to guarantee safety and efficacy of the applied therapy. Fine-tuning of the formulation is used to obtain optimal stability, because already small changes in the nature and/or concentration of the ingredients of the formulation influence the antibody's stability.

Vedolizumab is a known therapeutic monoclonal antibody which is used for the treatment of ulcerative colitis and Crohn's disease. It binds to and inhibits integrin α4β7 (LPAM-1) which results anti-inflammatory activity in the gut. Vedolizumab is commonly stored in a lyophilized formulation which is reconstituted prior to administration. The marketed drug product Entyvio comprises a lyophilized Vedolizumab formulation for intravenous administration which is reconstituted to consisting of 60 mg/ml antibody, 50 mM histidine, 10 % (w/v) sucrose, 125 mM arginine HCl and 0.06 % (w/v) polysorbate 80 at pH 6.3 (see marketing authorization documents of Entyvio). A Vedolizumab formulation for subcutaneous administration is taught in WO 2012/151247 A2 and consists of 160 mg/ml antibody, 50 mM histidine, 125 mM arginine, 25 mM citrate and 0.2% polysorbate 80 at pH 6.5.

There is a need in the art for a Vedolizumab formulation for subcutaneous administration with optimized storage abilities.

### SUMMARY OF THE INVENTION

The present invention is based on the finding that the stability of the known subcutaneous Vedolizumab formulation can further be increased by optimizing its composition. Especially, removing citrate and adding sorbitol resulted in an improved formulation. The formulation according to the present invention in particular shows higher storage stability with lower tendency to form aggregates. In addition, also the amount of charged variants and succinimide intermediates is reduced when using the improved formulation.

The present invention therefore provides in a first aspect a pharmaceutical formulation comprising Vedolizumab, histidine, arginine, and sorbitol.

The present invention further provides in a second aspect a sealed container containing the pharmaceutical formulation according to the first aspect, and in a third aspect an article of manufacture comprising the container according to the second aspect.

In a fourth aspect, the present invention provides the pharmaceutical formulation according to the first aspect or the article of manufacture according to the third aspect for use in the treatment of inflammatory bowel disease such as ulcerative colitis, Crohn's disease and pouchitis, or of graft versus host disease.

Other objects, features, advantages and aspects of the present invention will become apparent to those skilled in the art from the following description and appended claims. It should be understood, however, that the following description, appended claims, and specific examples, which indicate preferred embodiments of the application, are given by way of illustration only. Various changes and modifications within the spirit and scope of the disclosed invention will become readily apparent to those skilled in the art from reading the following.

### DEFINITIONS

As used herein, the following expressions are generally intended to preferably have the meanings as set forth below, except to the extent that the context in which they are used indicates otherwise.

The expression "comprise", as used herein, besides its literal meaning also includes and specifically refers to the expressions "consist essentially of" and "consist of". Thus, the expression "comprise" refers to embodiments wherein the subject-matter which "comprises" specifically listed elements does not comprise further elements as well as embodiments wherein the subject-matter which "comprises" specifically listed elements may and/or indeed does encompass further elements. Likewise, the expression "have" is to be understood as the expression "comprise", also including and specifically referring to the expressions "consist essentially of" and "consist of". The term "consist essentially of", where possible, in particular refers to embodiments wherein the subject-matter comprises 20% or less, in particular 15% or less, 10% or less or especially 5% or less further elements in addition to the specifically listed elements of which the subject-matter consists essentially of.

The term "about", as used herein, is intended to provide flexibility to a specific value or a numerical range endpoint, providing that a given value may be "a little above" or "a little below" the indicated value accounting for variations one might see in the measurements taken among different instruments, samples, and sample preparations. The term usually means within 5%, and preferably within 1% of a given value or range. The term "about" also includes and specifically refers to the exact indicated number or range.

The term "antibody" in particular refers to a protein comprising at least two heavy chains and two light chains connected by disulfide bonds. Each heavy chain is comprised of a heavy chain variable region (VH) and a heavy chain constant region (CH). Each light chain is comprised of a light chain variable region (VL) and a light chain constant region (CL). The heavy chain-constant region comprises three heavy chain-constant domains (CH1, CH2 and CH3) wherein the first constant domain CH1 is adjacent to the variable region and is connected to the second constant domain CH2 by a hinge region. The light chain-constant region consists only of one constant domain. The variable regions can be further subdivided into regions of hypervariability, termed complementarity determining regions (CDRs), interspersed with regions that are more conserved, termed framework regions (FR), wherein each variable region comprises three CDRs and four FRs. The variable regions of the heavy and light chains contain a binding domain that interacts with an antigen.

The term "pharmaceutical formulation" particularly refers to a composition suitable for administering to a human or animal. Hence, the components of a pharmaceutical formulation are pharmaceutically acceptable.

The term "patient" means according to the invention a human being, a nonhuman primate or another animal, in particular a mammal such as a cow, horse, pig, sheep, goat, dog, cat or a rodent such as a mouse and rat. In a particularly preferred embodiment, the patient is a human being.

### DETAILED DESCRIPTION OF THE INVENTION

Based on stability tests of various different formulations for Vedolizumab, the present inventors have found that the claimed formulations, such as for example formulations comprising about 100 mM sorbitol, have optimal shelf storage stability in liquid form. Especially, the pharmaceutical composition shows a reduced formation of antibody aggregates, charged variants and succinimide intermediates.

In view of this, the present invention provides in a first aspect a pharmaceutical formulation comprising Vedolizumab, histidine, arginine, and sorbitol.

The pharmaceutical composition in particular is a liquid composition, especially an aqueous composition. Preferably, the pharmaceutical composition is suitable for subcutaneous administration to a human being.

### 1. Components of the pharmaceutical formulation

Vedolizumab is a humanized monoclonal IgG1 antibody which specifically binds to the integrin α4β7 dimer. Vedolizumab in particular comprises a heavy chain amino acid sequence of SEQ ID NO: 1 and a light chain amino acid sequence of SEQ ID NO: 2. In particular, Vedolizumab is the antibody defined in the WHO Drug Information, Vol. 22, No. 4, 2008 with the International Nonproprietary Name (INN) "Vedolizumab". Vedolizumab may be recombinantly produced in various host cells and suitable cells for recombinant antibody production are known in the art. In one embodiment, Vedolizumab was recombinantly produced in a mammalian cell. Suitable mammalian cells are known in the art and comprise rodent as well as human cell lines. In one embodiment, Vedolizumab was recombinantly produced in a hamster cell. In one embodiment, Vedolizumab was recombinantly produced in a CHO cell. In certain embodiments, the concentration of Vedolizumab in the pharmaceutical formulation is in the range of 150 to 170 mg/ml, preferably about 160 mg/ml, such as 158.8 mg/ml.

Histidine may be present in the pharmaceutical formulation in form of the free base or as acid addition salt. In particular, histidine is present in the pharmaceutical formulation as a mixture of free base and acid addition salt. In certain embodiments, histidine is used as buffer substance and the ratio of free base and acid addition salt is chosen so as to adjust the pharmaceutical formulation to the desired pH value. In certain embodiments, the acid addition salt of histidine may in particular be histidine HCl. In particular, the L-enantiomer of histidine is used, i.e. L-histidine. In certain embodiments, histidine is present in the pharmaceutical formulation as a mixture of L-histidine and L-histidine HCl. In certain embodiments, the concentration of histidine in the pharmaceutical formulation is in the range of 20 to 100 mM, preferably about 50 mM.

Arginine may be present in the pharmaceutical formulation or used for its preparation in form of the free base or as acid addition salt. In certain embodiments, arginine is present or used as arginine HCl. In particular, the L-enantiomer of arginine is used, i.e. L-arginine. In certain embodiments, the concentration of arginine in the pharmaceutical formulation is in the range of 150 to 200 mM, preferably about 180 mM.

Sorbitol may be present in the pharmaceutical formulation in a concentration in the range of 50 to 200 mM, preferably about 100 mM.

The pharmaceutical formulation in particular has a neutral to light acidic pH. In certain embodiments, the pH of the pharmaceutical formulation is between 6 and 7. Preferably, the pH of the pharmaceutical formulation is about 6.5.

In certain embodiments, the pharmaceutical formulation further comprises a surfactant. The surfactant may especially be a non-ionic surfactant, for example a polymeric non-ionic surfactant. Suitable examples of surfactants are poloxamers. In certain embodiments, the surfactant is a poloxamer, such as poloxamers 188 and 407, especially poloxamer 188. Poloxamer 188, also known as Pluronic F68, is a nonionic triblock copolymers composed of a central hydrophobic chain of polyoxypropylene flanked by two hydrophilic chains of polyoxyethylene. In particular, poloxamer 188 has an oxyethylene content of about 80% and an average molecular weight of about 8400 Da. In certain embodiments, the concentration of the surfactant in the pharmaceutical formulation is in the range of 0.01 to 1% (w/v). In embodiments wherein the surfactant is poloxamer 188, the concentration of the surfactant in the pharmaceutical formulation is in particular in the range of 0.02 to 0.1% (w/v), preferably about 0.06% (w/v).

In specific embodiments, the pharmaceutical formulation consists of water, about 158.8 mg/ml Vedolizumab, about 100 mM sorbitol, about 180 mM arginine, about 50 mM histidine and about 0.06% (w/v) poloxamer 188, and has a pH of about 6.5, wherein "about" refers to a variation by +/- 5% of the indicated value.

In certain embodiments, the pharmaceutical formulation does not comprise citrate. In specific embodiments, the pharmaceutical formulation does not comprise any antioxidant and/or chelator.

In specific embodiments, the pharmaceutical formulation is an aqueous pharmaceutical formulation. In specific embodiments, the pharmaceutical formulation consists of Vedolizumab, arginine, histidine, sorbitol, a surfactant, and water, optionally including suitable counterions.

### 2. Use and preparation of the pharmaceutical formulation

The pharmaceutical formulation is advantageously suitable for parenteral delivery. Parenteral administration includes e.g. subcutaneous, intramuscular, intradermal, intramedullary, intrathecal, direct intraventricular, intravenous, intraperitoneal and intravitreal injections. In one embodiment, the pharmaceutical formulation is an injectable formulation. In certain embodiments, the pharmaceutical formulation is suitable for subcutaneous, intravenous, or intramuscular administration. Preferably, the pharmaceutical formulation is suitable for subcutaneous injection.

The pharmaceutical formulation can be prepared by providing a purified Vedolizumab composition and exchanging the buffer using well-known methods. The buffer exchanging may for example be the last step of a purification process of Vedolizumab. Vedolizumab in the final buffer may be concentrated to a desired concentration or a more concentrated form of the antibody is diluted to achieve the desired concentration. Concentration of the formulation can be carried out by any suitable method. In one aspect, the concentration process can include ultrafiltration.

The pharmaceutical formulation can alternatively be prepared by providing a purified Vedolizumab composition comprising some, but not all of the components described herein, and adding the missing components to the composition. In particular, the purified Vedolizumab composition may be a stock solution having a higher concentration than the final pharmaceutical formulation and the missing components may be present in one or more further stock solution. Upon mixing these stock solutions, the pharmaceutical formulation having the desired concentrations of the components is obtained.

The pharmaceutical formulation can also be prepared by reconstitution of a suitable lyophilized formulation. Reconstitution in particular is performed by adding water to the lyophilized formulation.

### 3. Specific embodiments of the pharmaceutical formulation

In specific embodiments, the pharmaceutical formulation comprises 150 to 170 mg/ml Vedolizumab, 50 to 200 mM sorbitol, 150 to 200 mM arginine and 20 to 100 mM histidine, and has a pH in the range of 6 to 7.

In specific embodiments, the pharmaceutical formulation comprises about 158.8 mg/ml Vedolizumab, 50 to 200 mM sorbitol, 150 to 200 mM arginine and 20 to 100 mM histidine, and has a pH in the range of 6 to 7.

In specific embodiments, the pharmaceutical formulation comprises 150 to 170 mg/ml Vedolizumab, 50 to 200 mM sorbitol, about 180 mM arginine and 20 to 100 mM histidine, and has a pH in the range of 6 to 7.

In specific embodiments, the pharmaceutical formulation comprises 150 to 170 mg/ml Vedolizumab, 50 to 200 mM sorbitol, 150 to 200 mM arginine and about 50 mM histidine, and has a pH in the range of 6 to 7.

In specific embodiments, the pharmaceutical formulation comprises 150 to 170 mg/ml Vedolizumab, 50 to 200 mM sorbitol, 150 to 200 mM arginine and 20 to 100 mM histidine, and has a pH of about 6.5.

In specific embodiments, the pharmaceutical formulation comprises about 158.8 mg/ml Vedolizumab, 50 to 200 mM sorbitol, about 180 mM arginine and about 50 mM histidine, and has a pH in the range of 6 to 7.

In specific embodiments, the pharmaceutical formulation comprises about 158.8 mg/ml Vedolizumab, 50 to 200 mM sorbitol, about 180 mM arginine and about 50 mM histidine, and has a pH of about 6.5.

In specific embodiments, the pharmaceutical formulation comprises 150 to 170 mg/ml Vedolizumab, 50 to 200 mM sorbitol, 150 to 200 mM arginine, 20 to 100 mM histidine and 0.02 to 0.1% (w/v) poloxamer 188, and has a pH in the range of 6 to 7.

In specific embodiments, the pharmaceutical formulation comprises about 158.8 mg/ml Vedolizumab, 50 to 200 mM sorbitol, 150 to 200 mM arginine, 20 to 100 mM histidine and 0.02 to 0.1% (w/v) poloxamer 188, and has a pH in the range of 6 to 7.

In specific embodiments, the pharmaceutical formulation comprises 150 to 170 mg/ml Vedolizumab, 50 to 200 mM sorbitol, about 180 mM arginine, 20 to 100 mM histidine and 0.02 to 0.1% (w/v) poloxamer 188, and has a pH in the range of 6 to 7.

In specific embodiments, the pharmaceutical formulation comprises 150 to 170 mg/ml Vedolizumab, 50 to 200 mM sorbitol, 150 to 200 mM arginine, about 50 mM histidine and 0.02 to 0.1% (w/v) poloxamer 188, and has a pH in the range of 6 to 7.

In specific embodiments, the pharmaceutical formulation comprises 150 to 170 mg/ml Vedolizumab, 50 to 200 mM sorbitol, 150 to 200 mM arginine, 20 to 100 mM histidine and 0.02 to 0.1% (w/v) poloxamer 188, and has a pH of about 6.5.

In specific embodiments, the pharmaceutical formulation comprises about 158.8 mg/ml Vedolizumab, 50 to 200 mM sorbitol, about 180 mM arginine, about 50 mM histidine and 0.02 to 0.1% (w/v) poloxamer 188, and has a pH in the range of 6 to 7.

In specific embodiments, the pharmaceutical formulation comprises about 158.8 mg/ml Vedolizumab, 50 to 200 mM sorbitol, about 180 mM arginine, about 50 mM histidine and 0.02 to 0.1% (w/v) poloxamer 188, and has a pH of about 6.5.

In specific embodiments, the pharmaceutical formulation comprises 150 to 170 mg/ml Vedolizumab, 50 to 200 mM sorbitol, 150 to 200 mM arginine, 20 to 100 mM histidine and 0.02 to 0.1% (w/v) poloxamer 188, and has a pH in the range of 6 to 7.

In specific embodiments, the pharmaceutical formulation comprises about 158.8 mg/ml Vedolizumab, 50 to 200 mM sorbitol, 150 to 200 mM arginine, 20 to 100 mM histidine and 0.02 to 0.1% (w/v) poloxamer 188, and has a pH in the range of 6 to 7.

In specific embodiments, the pharmaceutical formulation comprises 150 to 170 mg/ml Vedolizumab, 50 to 200 mM sorbitol, about 180 mM arginine, 20 to 100 mM histidine and 0.02 to 0.1% (w/v) poloxamer 188, and has a pH in the range of 6 to 7.

In specific embodiments, the pharmaceutical formulation comprises 150 to 170 mg/ml Vedolizumab, 50 to 200 mM sorbitol, 150 to 200 mM arginine, about 50 mM histidine and 0.02 to 0.1% (w/v) poloxamer 188, and has a pH in the range of 6 to 7.

In specific embodiments, the pharmaceutical formulation comprises 150 to 170 mg/ml Vedolizumab, 50 to 200 mM sorbitol, 150 to 200 mM arginine, 20 to 100 mM histidine and 0.02 to 0.1% (w/v) poloxamer 188, and has a pH of about 6.5.

In specific embodiments, the pharmaceutical formulation comprises about 158.8 mg/ml Vedolizumab, 50 to 200 mM sorbitol, about 180 mM arginine, about 50 mM histidine and 0.02 to 0.1% (w/v) poloxamer 188, and has a pH in the range of 6 to 7.

In specific embodiments, the pharmaceutical formulation comprises about 158.8 mg/ml Vedolizumab, 50 to 200 mM sorbitol, about 180 mM arginine, about 50 mM histidine and 0.02 to 0.1% (w/v) poloxamer 188, and has a pH of about 6.5.

In specific embodiments, the pharmaceutical formulation comprises 150 to 170 mg/ml Vedolizumab, 50 to 200 mM sorbitol, 150 to 200 mM arginine, 20 to 100 mM histidine and about 0.06% (w/v) poloxamer 188, and has a pH in the range of 6 to 7.

In specific embodiments, the pharmaceutical formulation comprises about 158.8 mg/ml Vedolizumab, 50 to 200 mM sorbitol, 150 to 200 mM arginine, 20 to 100 mM histidine and about 0.06% (w/v) poloxamer 188, and has a pH in the range of 6 to 7.

In specific embodiments, the pharmaceutical formulation comprises 150 to 170 mg/ml Vedolizumab, 50 to 200 mM sorbitol, about 180 mM arginine, 20 to 100 mM histidine and about 0.06% (w/v) poloxamer 188, and has a pH in the range of 6 to 7.

In specific embodiments, the pharmaceutical formulation comprises 150 to 170 mg/ml Vedolizumab, 50 to 200 mM sorbitol, 150 to 200 mM arginine, about 50 mM histidine and about 0.06% (w/v) poloxamer 188, and has a pH in the range of 6 to 7.

In specific embodiments, the pharmaceutical formulation comprises 150 to 170 mg/ml Vedolizumab, 50 to 200 mM sorbitol, 150 to 200 mM arginine, 20 to 100 mM histidine and about 0.06% (w/v) poloxamer 188, and has a pH of about 6.5.

In specific embodiments, the pharmaceutical formulation comprises about 158.8 mg/ml Vedolizumab, 50 to 200 mM sorbitol, about 180 mM arginine, about 50 mM histidine and about 0.06% (w/v) poloxamer 188, and has a pH in the range of 6 to 7.

In specific embodiments, the pharmaceutical formulation comprises about 158.8 mg/ml Vedolizumab, 50 to 200 mM sorbitol, about 180 mM arginine, about 50 mM histidine and about 0.06% (w/v) poloxamer 188, and has a pH of about 6.5.

The concentration of sorbitol in the above specific embodiments may in particular be 80 to 120 mM, preferably about 100 mM.

The pharmaceutical formulation may in particular consist of water and the components described in any one of the above specific embodiments, and have the pH described in said specific embodiment. The concentration of sorbitol in these embodiments may in particular be 80 to 120 mM, preferably about 100 mM.

In specific embodiments, the pharmaceutical formulation comprises about 158.8 mg/ml Vedolizumab, 80 to 120 mM sorbitol, about 180 mM arginine, about 50 mM histidine and about 0.06% (w/v) poloxamer 188, and has a pH of about 6.5.

In specific embodiments, the pharmaceutical formulation comprises about 158.8 mg/ml Vedolizumab, about 100 mM sorbitol, about 180 mM arginine, about 50 mM histidine and about 0.06% (w/v) poloxamer 188, and has a pH of about 6.5.

In specific embodiments, the pharmaceutical formulation consists of water, 150 to 170 mg/ml Vedolizumab, 50 to 200 mM sorbitol, 150 to 200 mM arginine, 20 to 100 mM histidine and 0.02 to 0.1% (w/v) poloxamer 188, and has a pH in the range of 6 to 7.

In specific embodiments, the pharmaceutical formulation consists of water, about 158.8 mg/ml Vedolizumab, 80 to 120 mM sorbitol, about 180 mM arginine, about 50 mM histidine and about 0.06% (w/v) poloxamer 188, and has a pH of about 6.5.

In specific embodiments, the liquid pharmaceutical formulation consists of water, about 158.8 mg/ml Vedolizumab, about 100 mM sorbitol, about 180 mM arginine, about 50 mM histidine and about 0.06% (w/v) poloxamer 188, and has a pH of about 6.5.

In certain embodiments, the pharmaceutical formulation comprises the following components:

**Table 1: Specific embodiments of the pharmaceutical formulation**

| **Vedo [mg/ml]** | **sorbitol [mM]** | **arginine [mM]** | **histidine [mM]** | **PX 188 [% (w/v)]** | **pH** |
|---|---|---|---|---|---|
| 150 - 170 | 50 - 200 | 150 - 200 | 20 - 100 | 0.02 - 0.1 | 6 - 7 |
| 150 - 170 | 80 - 120 | 150 - 200 | 20 - 100 | 0.02 - 0.1 | 6 - 7 |
| 150 - 170 | 100 | 150 - 200 | 20 - 100 | 0.02 - 0.1 | 6 - 7 |
| 158.8 | 50 - 200 | 150 - 200 | 20 - 100 | 0.02 - 0.1 | 6 - 7 |
| 158.8 | 80 - 120 | 150 - 200 | 20 - 100 | 0.02 - 0.1 | 6 - 7 |
| 158.8 | 100 | 150 - 200 | 20 - 100 | 0.02 - 0.1 | 6 - 7 |
| 150 - 170 | 50 - 200 | 180 | 20 - 100 | 0.02 - 0.1 | 6 - 7 |
| 150 - 170 | 80 - 120 | 180 | 20 - 100 | 0.02 - 0.1 | 6 - 7 |
| 150 - 170 | 100 | 180 | 20 - 100 | 0.02 - 0.1 | 6 - 7 |
| 150 - 170 | 50 - 200 | 150 - 200 | 50 | 0.02 - 0.1 | 6 - 7 |
| 150 - 170 | 80 - 120 | 150 - 200 | 50 | 0.02 - 0.1 | 6 - 7 |
| 150 - 170 | 100 | 150 - 200 | 50 | 0.02 - 0.1 | 6 - 7 |
| 150 - 170 | 50 - 200 | 150 - 200 | 20 - 100 | 0.06 | 6 - 7 |
| 150 - 170 | 80 - 120 | 150 - 200 | 20 - 100 | 0.06 | 6 - 7 |
| 150 - 170 | 100 | 150 - 200 | 20 - 100 | 0.06 | 6 - 7 |
| 150 - 170 | 50 - 200 | 150 - 200 | 20 - 100 | 0.02 - 0.1 | 6.5 |
| 150 - 170 | 80 - 120 | 150 - 200 | 20 - 100 | 0.02 - 0.1 | 6.5 |
| 150 - 170 | 100 | 150 - 200 | 20 - 100 | 0.02 - 0.1 | 6.5 |
| 158.8 | 50 - 200 | 180 | 20 - 100 | 0.02 - 0.1 | 6 - 7 |
| 158.8 | 80 - 120 | 180 | 20 - 100 | 0.02 - 0.1 | 6 - 7 |
| 158.8 | 100 | 180 | 20 - 100 | 0.02 - 0.1 | 6 - 7 |
| 158.8 | 50 - 200 | 150 - 200 | 50 | 0.02 - 0.1 | 6 - 7 |
| 158.8 | 80 - 120 | 150 - 200 | 50 | 0.02 - 0.1 | 6 - 7 |
| 158.8 | 100 | 150 - 200 | 50 | 0.02 - 0.1 | 6 - 7 |
| 158.8 | 50 - 200 | 150 - 200 | 20 - 100 | 0.06 | 6 - 7 |
| 158.8 | 80 - 120 | 150 - 200 | 20 - 100 | 0.06 | 6 - 7 |
| 158.8 | 100 | 150 - 200 | 20 - 100 | 0.06 | 6 - 7 |
| 158.8 | 50 - 200 | 150 - 200 | 20 - 100 | 0.02 - 0.1 | 6.5 |
| 158.8 | 80 - 120 | 150 - 200 | 20 - 100 | 0.02 - 0.1 | 6.5 |
| 158.8 | 100 | 150 - 200 | 20 - 100 | 0.02 - 0.1 | 6.5 |
| 150 - 170 | 50 - 200 | 180 | 50 | 0.02 - 0.1 | 6 - 7 |
| 150 - 170 | 80 - 120 | 180 | 50 | 0.02 - 0.1 | 6 - 7 |
| 150 - 170 | 100 | 180 | 50 | 0.02 - 0.1 | 6 - 7 |
| 150 - 170 | 50 - 200 | 180 | 20 - 100 | 0.06 | 6 - 7 |
| 150 - 170 | 80 - 120 | 180 | 20 - 100 | 0.06 | 6 - 7 |
| 150 - 170 | 100 | 180 | 20 - 100 | 0.06 | 6 - 7 |
| 150 - 170 | 50 - 200 | 180 | 20 - 100 | 0.02 - 0.1 | 6.5 |
| 150 - 170 | 80 - 120 | 180 | 20 - 100 | 0.02 - 0.1 | 6.5 |
| 150 - 170 | 100 | 180 | 20 - 100 | 0.02 - 0.1 | 6.5 |
| 150 - 170 | 50 - 200 | 150 - 200 | 50 | 0.06 | 6 - 7 |
| 150 - 170 | 80 - 120 | 150 - 200 | 50 | 0.06 | 6 - 7 |
| 150 - 170 | 100 | 150 - 200 | 50 | 0.06 | 6 - 7 |
| 150 - 170 | 50 - 200 | 150 - 200 | 50 | 0.02 - 0.1 | 6.5 |
| 150 - 170 | 80 - 120 | 150 - 200 | 50 | 0.02 - 0.1 | 6.5 |
| 150 - 170 | 100 | 150 - 200 | 50 | 0.02 - 0.1 | 6.5 |
| 150 - 170 | 50 - 200 | 150 - 200 | 20 - 100 | 0.06 | 6.5 |
| 150 - 170 | 80 - 120 | 150 - 200 | 20 - 100 | 0.06 | 6.5 |
| 150 - 170 | 100 | 150 - 200 | 20 - 100 | 0.06 | 6.5 |
| 158.8 | 50 - 200 | 180 | 50 | 0.02 - 0.1 | 6 - 7 |
| 158.8 | 80 - 120 | 180 | 50 | 0.02 - 0.1 | 6 - 7 |
| 158.8 | 100 | 180 | 50 | 0.02 - 0.1 | 6 - 7 |
| 158.8 | 50 - 200 | 180 | 20 - 100 | 0.06 | 6 - 7 |
| 158.8 | 80 - 120 | 180 | 20 - 100 | 0.06 | 6 - 7 |
| 158.8 | 100 | 180 | 20 - 100 | 0.06 | 6 - 7 |
| 158.8 | 50 - 200 | 180 | 20 - 100 | 0.02 - 0.1 | 6.5 |
| 158.8 | 80 - 120 | 180 | 20 - 100 | 0.02 - 0.1 | 6.5 |
| 158.8 | 100 | 180 | 20 - 100 | 0.02 - 0.1 | 6.5 |
| 158.8 | 50 - 200 | 150 - 200 | 50 | 0.06 | 6 - 7 |
| 158.8 | 80 - 120 | 150 - 200 | 50 | 0.06 | 6 - 7 |
| 158.8 | 100 | 150 - 200 | 50 | 0.06 | 6 - 7 |
| 158.8 | 50 - 200 | 150 - 200 | 50 | 0.02 - 0.1 | 6.5 |
| 158.8 | 80 - 120 | 150 - 200 | 50 | 0.02 - 0.1 | 6.5 |
| 158.8 | 100 | 150 - 200 | 50 | 0.02 - 0.1 | 6.5 |
| 158.8 | 50 - 200 | 150 - 200 | 20 - 100 | 0.06 | 6.5 |
| 158.8 | 80 - 120 | 150 - 200 | 20 - 100 | 0.06 | 6.5 |
| 158.8 | 100 | 150 - 200 | 20 - 100 | 0.06 | 6.5 |
| 150 - 170 | 50 - 200 | 180 | 50 | 0.06 | 6 - 7 |
| 150 - 170 | 80 - 120 | 180 | 50 | 0.06 | 6 - 7 |
| 150 - 170 | 100 | 180 | 50 | 0.06 | 6 - 7 |
| 150 - 170 | 50 - 200 | 180 | 50 | 0.02 - 0.1 | 6.5 |
| 150 - 170 | 80 - 120 | 180 | 50 | 0.02 - 0.1 | 6.5 |
| 150 - 170 | 100 | 180 | 50 | 0.02 - 0.1 | 6.5 |
| 150 - 170 | 50 - 200 | 150 - 200 | 50 | 0.06 | 6.5 |
| 150 - 170 | 80 - 120 | 150 - 200 | 50 | 0.06 | 6.5 |
| 150 - 170 | 100 | 150 - 200 | 50 | 0.06 | 6.5 |
| 158.8 | 50 - 200 | 180 | 50 | 0.06 | 6 - 7 |
| 158.8 | 80 - 120 | 180 | 50 | 0.06 | 6 - 7 |
| 158.8 | 100 | 180 | 50 | 0.06 | 6 - 7 |
| 158.8 | 50 - 200 | 180 | 50 | 0.02 - 0.1 | 6.5 |
| 158.8 | 80 - 120 | 180 | 50 | 0.02 - 0.1 | 6.5 |
| 158.8 | 100 | 180 | 50 | 0.02 - 0.1 | 6.5 |
| 158.8 | 50 - 200 | 180 | 20 - 100 | 0.06 | 6.5 |
| 158.8 | 80 - 120 | 180 | 20 - 100 | 0.06 | 6.5 |
| 158.8 | 100 | 180 | 20 - 100 | 0.06 | 6.5 |
| 158.8 | 50 - 200 | 150 - 200 | 50 | 0.06 | 6.5 |
| 158.8 | 80 - 120 | 150 - 200 | 50 | 0.06 | 6.5 |
| 158.8 | 100 | 150 - 200 | 50 | 0.06 | 6.5 |
| 150 - 170 | 50 - 200 | 180 | 50 | 0.06 | 6.5 |
| 150 - 170 | 80 - 120 | 180 | 50 | 0.06 | 6.5 |
| 150 - 170 | 100 | 180 | 50 | 0.06 | 6.5 |
| 158.8 | 50 - 200 | 180 | 50 | 0.06 | 6.5 |
| 158.8 | 80 - 120 | 180 | 50 | 0.06 | 6.5 |
| 158.8 | 100 | 180 | 50 | 0.06 | 6.5 |

| | | | | | |
|---|---|---|---|---|---|
| Vedo: Vedolizumab; PX 188: poloxamer 188 | | | | | |

In certain embodiments, the pharmaceutical formulation consists of the following components in addition to water:

**Table 2: Specific embodiments of the pharmaceutical formulation**

| **Vedo [mg/ml]** | **sorbitol [mM]** | **arginine [mM]** | **histidine [mM]** | **PX 188 [% (w/v)]** | **pH** |
|---|---|---|---|---|---|
| 150 - 170 | 50 - 200 | 150 - 200 | 20 - 100 | 0.02 - 0.1 | 6 - 7 |
| 150 - 170 | 80 - 120 | 150 - 200 | 20 - 100 | 0.02 - 0.1 | 6 - 7 |
| 150 - 170 | 100 | 150 - 200 | 20 - 100 | 0.02 - 0.1 | 6 - 7 |
| 158.8 | 50 - 200 | 150 - 200 | 20 - 100 | 0.02 - 0.1 | 6 - 7 |
| 158.8 | 80 - 120 | 150 - 200 | 20 - 100 | 0.02 - 0.1 | 6 - 7 |
| 158.8 | 100 | 150 - 200 | 20 - 100 | 0.02 - 0.1 | 6 - 7 |
| 150 - 170 | 50 - 200 | 180 | 20 - 100 | 0.02 - 0.1 | 6 - 7 |
| 150 - 170 | 80 - 120 | 180 | 20 - 100 | 0.02 - 0.1 | 6 - 7 |
| 150 - 170 | 100 | 180 | 20 - 100 | 0.02 - 0.1 | 6 - 7 |
| 150 - 170 | 50 - 200 | 150 - 200 | 50 | 0.02 - 0.1 | 6 - 7 |
| 150 - 170 | 80 - 120 | 150 - 200 | 50 | 0.02 - 0.1 | 6 - 7 |
| 150 - 170 | 100 | 150 - 200 | 50 | 0.02 - 0.1 | 6 - 7 |
| 150 - 170 | 50 - 200 | 150 - 200 | 20 - 100 | 0.06 | 6 - 7 |
| 150 - 170 | 80 - 120 | 150 - 200 | 20 - 100 | 0.06 | 6 - 7 |
| 150 - 170 | 100 | 150 - 200 | 20 - 100 | 0.06 | 6 - 7 |
| 150 - 170 | 50 - 200 | 150 - 200 | 20 - 100 | 0.02 - 0.1 | 6.5 |
| 150 - 170 | 80 - 120 | 150 - 200 | 20 - 100 | 0.02 - 0.1 | 6.5 |
| 150 - 170 | 100 | 150 - 200 | 20 - 100 | 0.02 - 0.1 | 6.5 |
| 158.8 | 50 - 200 | 180 | 20 - 100 | 0.02 - 0.1 | 6 - 7 |
| 158.8 | 80 - 120 | 180 | 20 - 100 | 0.02 - 0.1 | 6 - 7 |
| 158.8 | 100 | 180 | 20 - 100 | 0.02 - 0.1 | 6 - 7 |
| 158.8 | 50 - 200 | 150 - 200 | 50 | 0.02 - 0.1 | 6 - 7 |
| 158.8 | 80 - 120 | 150 - 200 | 50 | 0.02 - 0.1 | 6 - 7 |
| 158.8 | 100 | 150 - 200 | 50 | 0.02 - 0.1 | 6 - 7 |
| 158.8 | 50 - 200 | 150 - 200 | 20 - 100 | 0.06 | 6 - 7 |
| 158.8 | 80 - 120 | 150 - 200 | 20 - 100 | 0.06 | 6 - 7 |
| 158.8 | 100 | 150 - 200 | 20 - 100 | 0.06 | 6 - 7 |
| 158.8 | 50 - 200 | 150 - 200 | 20 - 100 | 0.02 - 0.1 | 6.5 |
| 158.8 | 80 - 120 | 150 - 200 | 20 - 100 | 0.02 - 0.1 | 6.5 |
| 158.8 | 100 | 150 - 200 | 20 - 100 | 0.02 - 0.1 | 6.5 |
| 150 - 170 | 50 - 200 | 180 | 50 | 0.02 - 0.1 | 6 - 7 |
| 150 - 170 | 80 - 120 | 180 | 50 | 0.02 - 0.1 | 6 - 7 |
| 150 - 170 | 100 | 180 | 50 | 0.02 - 0.1 | 6 - 7 |
| 150 - 170 | 50 - 200 | 180 | 20 - 100 | 0.06 | 6 - 7 |
| 150 - 170 | 80 - 120 | 180 | 20 - 100 | 0.06 | 6 - 7 |
| 150 - 170 | 100 | 180 | 20 - 100 | 0.06 | 6 - 7 |
| 150 - 170 | 50 - 200 | 180 | 20 - 100 | 0.02 - 0.1 | 6.5 |
| 150 - 170 | 80 - 120 | 180 | 20 - 100 | 0.02 - 0.1 | 6.5 |
| 150 - 170 | 100 | 180 | 20 - 100 | 0.02 - 0.1 | 6.5 |
| 150 - 170 | 50 - 200 | 150 - 200 | 50 | 0.06 | 6 - 7 |
| 150 - 170 | 80 - 120 | 150 - 200 | 50 | 0.06 | 6 - 7 |
| 150 - 170 | 100 | 150 - 200 | 50 | 0.06 | 6 - 7 |
| 150 - 170 | 50 - 200 | 150 - 200 | 50 | 0.02 - 0.1 | 6.5 |
| 150 - 170 | 80 - 120 | 150 - 200 | 50 | 0.02 - 0.1 | 6.5 |
| 150 - 170 | 100 | 150 - 200 | 50 | 0.02 - 0.1 | 6.5 |
| 150 - 170 | 50 - 200 | 150 - 200 | 20 - 100 | 0.06 | 6.5 |
| 150 - 170 | 80 - 120 | 150 - 200 | 20 - 100 | 0.06 | 6.5 |
| 150 - 170 | 100 | 150 - 200 | 20 - 100 | 0.06 | 6.5 |
| 158.8 | 50 - 200 | 180 | 50 | 0.02 - 0.1 | 6 - 7 |
| 158.8 | 80 - 120 | 180 | 50 | 0.02 - 0.1 | 6 - 7 |
| 158.8 | 100 | 180 | 50 | 0.02 - 0.1 | 6 - 7 |
| 158.8 | 50 - 200 | 180 | 20 - 100 | 0.06 | 6 - 7 |
| 158.8 | 80 - 120 | 180 | 20 - 100 | 0.06 | 6 - 7 |
| 158.8 | 100 | 180 | 20 - 100 | 0.06 | 6 - 7 |
| 158.8 | 50 - 200 | 180 | 20 - 100 | 0.02 - 0.1 | 6.5 |
| 158.8 | 80 - 120 | 180 | 20 - 100 | 0.02 - 0.1 | 6.5 |
| 158.8 | 100 | 180 | 20 - 100 | 0.02 - 0.1 | 6.5 |
| 158.8 | 50 - 200 | 150 - 200 | 50 | 0.06 | 6 - 7 |
| 158.8 | 80 - 120 | 150 - 200 | 50 | 0.06 | 6 - 7 |
| 158.8 | 100 | 150 - 200 | 50 | 0.06 | 6 - 7 |
| 158.8 | 50 - 200 | 150 - 200 | 50 | 0.02 - 0.1 | 6.5 |
| 158.8 | 80 - 120 | 150 - 200 | 50 | 0.02 - 0.1 | 6.5 |
| 158.8 | 100 | 150 - 200 | 50 | 0.02 - 0.1 | 6.5 |
| 158.8 | 50 - 200 | 150 - 200 | 20 - 100 | 0.06 | 6.5 |
| 158.8 | 80 - 120 | 150 - 200 | 20 - 100 | 0.06 | 6.5 |
| 158.8 | 100 | 150 - 200 | 20 - 100 | 0.06 | 6.5 |
| 150 - 170 | 50 - 200 | 180 | 50 | 0.06 | 6 - 7 |
| 150 - 170 | 80 - 120 | 180 | 50 | 0.06 | 6 - 7 |
| 150 - 170 | 100 | 180 | 50 | 0.06 | 6 - 7 |
| 150 - 170 | 50 - 200 | 180 | 50 | 0.02 - 0.1 | 6.5 |
| 150 - 170 | 80 - 120 | 180 | 50 | 0.02 - 0.1 | 6.5 |
| 150 - 170 | 100 | 180 | 50 | 0.02 - 0.1 | 6.5 |
| 150 - 170 | 50 - 200 | 150 - 200 | 50 | 0.06 | 6.5 |
| 150 - 170 | 80 - 120 | 150 - 200 | 50 | 0.06 | 6.5 |
| 150 - 170 | 100 | 150 - 200 | 50 | 0.06 | 6.5 |
| 158.8 | 50 - 200 | 180 | 50 | 0.06 | 6 - 7 |
| 158.8 | 80 - 120 | 180 | 50 | 0.06 | 6 - 7 |
| 158.8 | 100 | 180 | 50 | 0.06 | 6 - 7 |
| 158.8 | 50 - 200 | 180 | 50 | 0.02 - 0.1 | 6.5 |
| 158.8 | 80 - 120 | 180 | 50 | 0.02 - 0.1 | 6.5 |
| 158.8 | 100 | 180 | 50 | 0.02 - 0.1 | 6.5 |
| 158.8 | 50 - 200 | 180 | 20 - 100 | 0.06 | 6.5 |
| 158.8 | 80 - 120 | 180 | 20 - 100 | 0.06 | 6.5 |
| 158.8 | 100 | 180 | 20 - 100 | 0.06 | 6.5 |
| 158.8 | 50 - 200 | 150 - 200 | 50 | 0.06 | 6.5 |
| 158.8 | 80 - 120 | 150 - 200 | 50 | 0.06 | 6.5 |
| 158.8 | 100 | 150 - 200 | 50 | 0.06 | 6.5 |
| 150 - 170 | 50 - 200 | 180 | 50 | 0.06 | 6.5 |
| 150 - 170 | 80 - 120 | 180 | 50 | 0.06 | 6.5 |
| 150 - 170 | 100 | 180 | 50 | 0.06 | 6.5 |
| 158.8 | 50 - 200 | 180 | 50 | 0.06 | 6.5 |
| 158.8 | 80 - 120 | 180 | 50 | 0.06 | 6.5 |
| 158.8 | 100 | 180 | 50 | 0.06 | 6.5 |

| | | | | | |
|---|---|---|---|---|---|
| Vedo: Vedolizumab; PX 188: poloxamer 188 | | | | | |

### 4. Products containing the pharmaceutical formulation

The present invention provides in a second aspect a sealed container containing the pharmaceutical formulation according to the first aspect of the present invention. In specific embodiments, the sealed contained is a vial, such as a glass vial, or a syringe.

In certain embodiments, the sealed container comprises a headspace, especially a headspace filled with nitrogen.

In certain embodiments, the sealed contained is a prefilled syringe. Prefilled syringes are devices that contain the pharmaceutical formulation, a needle for injection of the pharmaceutical formulation, and a means for ejecting the pharmaceutical formulation through the needle. Prefilled syringes may be designed to provide exactly one dose of the pharmaceutical formulation, or multiple doses. The prefilled syringe in particular may be an injector pen, an auto-injector, or a needle safety device. In preferred embodiments, the prefilled syringe is for subcutaneous administration of the pharmaceutical formulation. Prefilled syringes for subcutaneous administration in particular comprise a 25-35 gauge needle with a length of 3 to 20 mm. In certain embodiments, the prefilled syringe is a glass syringe.

The present invention provides in a third aspect an article of manufacture comprising the container according to the second aspect. The article of manufacture may further contain instructions for use such as a patient information leaflet.

### 5. Therapeutic uses of the pharmaceutical formulation

In a fourth aspect, the present invention provides the pharmaceutical formulation according to the first aspect of the present invention or the article of manufacture according to the third aspect of the present invention for use in the treatment of a disease. In specific embodiments, the disease is an inflammatory bowel disease such as ulcerative colitis, Crohn's disease and pouchitis. In further embodiments, the disease is graft versus host disease. The patient to be treated especially is a human patient. In certain embodiments, treatment of the disease involves subcutaneous administration of the pharmaceutical formulation.

The present invention provides a method of treatment of a patient in need thereof, comprising the step of subcutaneously administering to said patient a therapeutically effective amount of the pharmaceutical formulation according to the first aspect of the present invention. In specific embodiments, the disease is an inflammatory bowel disease such as ulcerative colitis, Crohn's disease and pouchitis. In further embodiments, the disease is graft versus host disease. The patient to be treated especially is a human patient.

### EXAMPLES

### Example 1: Test samples for short-term stability study

Tested formulations were aliquoted to 2R glass vials and stored at intended storage conditions (5°C±3°C), accelerated storage conditions (25°C±2°C / 60%±5% RH) and stress conditions (40°C±2°C / 75%±5% RH). The vials were stored in upright position to exclude possible influence of stopper on protein stability in tested formulations.

The compositions of the formulations tested in the study are presented in Table 3.

**Table 3: Overview of the composition of formulations F1 and F2.**

| **Formulation** | **Antibody** | **Buffer** | **pH** | **Additive** | **Excipient** | **Surfactant** |
|---|---|---|---|---|---|---|
| F1 | 160 mg/ml Vedolizumab | 50 mM histidine | 6.5 | 100 mM sorbitol | 180 mM arginine | 0.06% (w/v) PX188 |
| F2 | 160 mg/ml Vedolizumab | 50 mM L-histidine | 6.5 | 25 mM citric acid / sodium citrate | 124 mM L-arginine HCl | 0.2% (w/v) PS80 |

| | | | | | | |
|---|---|---|---|---|---|---|
| PS80: polysorbate 80; PX188: poloxamer 188 | | | | | | |

### Sample preparation

After UF/DF, an intermediate Vedolizumab solution (213.23 mg/mL antibody, 39.2 mM L-histidine, 97.8 mM L-arginine HCl, pH 6.53) was used to prepare all tested formulations. The protein solution was equilibrated to room temperature on the day of formulation preparation. After that, the bottle with protein solution was gently inverting several times for homogenization.

Investigated formulations were obtained by diluting the intermediate solution to ca. 160 mg/mL using excipients stock solutions (w/v). Each final composition was centrifugated at 9000 rpm for 5 minutes and filtered through a 0.22 µm filter into a sterile Nalgene^{™} bottle under laminar sterile air flow conditions. The prepared formulations were aliquoted (1 mL per vial) into 2R vials using serological pipette. After filling, the headspace in the vials was flushed with nitrogen for 5 s, and the vials were stoppered and crimped.

### Example 2: Test samples for long-term stability study

The tested formulation was aliquoted to sterile prefilled syringes in the volume of 0.76 mL per syringe and stored at intended storage conditions (5°C±3°C), accelerated storage conditions (25°C±2°C/ 60%±5% RH) and stress conditions (40°C±2°C/ 75%±5% RH). The prefilled syringes were stored in a horizontal position which provides the highest air/liquid and stopper/liquid interface area.

The samples tested in the study are presented in Table 4.

**Table 4: Overview of the composition of formulations F1 and the originator drug product (DP).**

| **Sample type** | **Antibody** | **Buffer** | **pH** | **Additive** | **Excipient** | **Surfactant** |
|---|---|---|---|---|---|---|
| F1 | 160 mg/ml Vedolizumab | 50 mM histidine | 6.5 | 100 mM sorbitol | 180 mM arginine | 0.06% (w/v) PX188 |
| Entyvio DP SC | 160 mg/ml Vedolizumab | 50 mM L-histidine | 6.5 | 25 mM citric acid / sodium citrate | 124 mM L-arginine HCl | 0.2% (w/v) PS80 |

| | | | | | | |
|---|---|---|---|---|---|---|
| PS80: polysorbate 80; PX188: poloxamer 188 | | | | | | |

### Sample preparation

An intermediate Vedolizumab solution (202.6 mg/mL antibody, 53.7 mM L-histidine, 210.1 mM L-arginine HCl, 0.07% (w/v) PX188, pH 6.30) was used to prepare the tested formulation.

The intermediate Vedolizumab solution was thawed at ambient conditions for 20 hours using a see-saw rocker (10 osc/min). After thawing, the content of the container was gently manually mixed for 5 minutes. Next, required portions were withdrawn from the container and diluted to 160 mg/ml antibody concentration using an appropriate solution. The prepared solution was filtered through a 0.22 µm sterile filter.

The prepared formulation was aliquoted to sterile prefilled syringes in the volume of 0.76 mL per syringe under a laminar flow hood. After aliquoting, the headspaces in the prefilled syringes were flushed with sterile nitrogen for 10 seconds. Afterwards, the prefilled syringes were stoppered using a manual stopper press. The headspace was set to approximately 5 mm height between solution and plunger when the syringe is held upright.

### Example 3: Stress / storage conditions of the liquid formulations

Samples were stored under the following conditions for up to three months (short-term stability study) or up to twelve months (long-term stability study):
1) Intended storage conditions, 5°C±3°C;
2) Accelerated conditions, 25°C±2°C / 60%±5% RH;
3) Stress conditions, 40°C±2°C / 75%±5% RH.

### Example 4: Size exclusion chromatography (SEC) for determination of antibody aggregation

### Method description

Size exclusion chromatography was performed with a Waters Acquity UPLC Protein BEH SEC Column (200Å, 1.7 µm, 4.6 mm x 150 mm), using a mobile phase of 100 mM sodium phosphate, 200 mM sodium chloride, pH 6.8, and a flow rate of 0.3 ml/min. Elution of the sample protein was detected by adsorption at 210 nm. A standard protein mix and a standard sample were used for calibration and system control. Peak areas were calculated by integration of the chromatogram using Empower Chromatography Data Software of Waters.

### Results

The results of SEC analysis did not show marked changes in the relative content of aggregates over the storage time for samples stored at 5°C. The samples stored at 25°C and 40°C showed gradual increase in aggregate content. Formulation F2 presented higher aggregates levels compared to formulation F1 in the short-term stability study conducted in vials.

**Table 5: Size exclusion chromatography of formulations F1 and F2.**

| storage conditions | total aggregates [%] | |
|---|---|---|
| | formulation F1 | formulation F2 |
| T0 | 0.27 | 0.29 |

| 5°C | | |
|---|---|---|
| T1M | 0.35 | 0.40 |
| T2M | 0.33 | 0.38 |
| T3M | 0.36 | 0.40 |

| 25°C | | |
|---|---|---|
| T1M | 0.45 | 0.50 |
| T2M | 0.53 | 0.57 |
| T3M | 0.61 | 0.64 |

| 40°C | | |
|---|---|---|
| T2W | 0.66 | 0.67 |
| T1M | 0.78 | 0.78 |
| T2M | 1.11 | 1.13 |
| T3M | 1.56 | 1.67 |

Entyvio DP SC presented higher aggregates levels compared to formulation F1 in the long-term stability study conducted in prefilled syringes. The upward trend in aggregate content is higher for Entyvio DP SC under accelerated and stressed conditions.

**Table 6: Size exclusion chromatography of formulations F1 and Entyvio DP SC.**

| storage conditions | total aggregates [%] | |
|---|---|---|
| | formulation F1 \| | Entyvio DP SC |
| T0 | 0.29 | 0.51 |

| 5°C | | |
|---|---|---|
| T6M | 0.45 | 0.68 |
| T12M | 0.68 | 0.73 |

| 25°C | | |
|---|---|---|
| T2M | 0.46 | 0.63 |
| T3M | 0.61 | 0.95 |
| T6M | 0.80 | 1.36 |

| 40°C | | |
|---|---|---|
| T2M | 0.97 | 1.35 |
| T3M | 1.43 | 2.36 |

### Example 5: Cation exchange chromatography (CEX) for determination of charged antibody variant formation

### Method description

Cation exchange chromatography was performed with a Waters Protein-Pak Hi Res SP column (7 µm, 4.6 mm x 100 mm). The chromatography was run with a flow rate of 0.7 ml/min using a mobile phase gradient from 72% A / 28% B to 37% A / 63% B with A being 20 mM MES, pH 6.5 and B being 20 mM MES, 0.1 M NaCl, pH 6.5. Elution of the sample protein was detected by adsorption at 280 nm. A standard sample was used for calibration and system control. Peak areas were calculated by integration of the chromatogram using Empower Chromatography Data Software of Waters.

### Results

A gradual decrease in main species content was observed at all three conditions. The decrease of main species reflected the changes in succinimide content observed in HIC analysis. Formulation F2 showed a lower percentage of main peak under accelerated and stress storage conditions compared to formulation F1 in the short-term stability study conducted in vials.

**Table 7: Cation exchange chromatography of formulations F1 and F2.**

| storage conditions | relative size of main peak [%] | |
|---|---|---|
| | formulation F1 | formulation F2 |
| T0 | 70.9 | 71.0 |

| 5°C | | |
|---|---|---|
| T1M | 71.2 | 70.7 |
| T2M | 70.7 | 70.5 |
| T3M | 70.4 | 70.6 |

| 25°C | | |
|---|---|---|
| T1M | 68.7 | 68.3 |
| T2M | 65.6 | 65.2 |
| T3M | 63.7 | 63.0 |

| 40°C | | |
|---|---|---|
| T2W | 59.6 | 59.5 |
| T1M | 52.4 | 52.0 |
| T2M | 35.5 | 34.1 |
| T3M | 26.1 | 25.0 |

Entyvio DP SC showed a lower percentage of main peak at all three conditions compared to formulation F1 in the long-term stability study conducted in prefilled syringes. The downward trend in main species content is higher for the Entyvio DP SC under intended and accelerated conditions.

**Table 8: Cation exchange chromatography of formulations F1 and Entyvio DP SC.**

| storage conditions | relative size of main peak [%] | |
|---|---|---|
| | formulation F1 | Entyvio DP SC |
| T0 | 72.1 | 65.6 |

| 5°C | | |
|---|---|---|
| T6M | 71.5 | 65.2 |
| T12M | 71.0 | 63.9 |

| 25°C | | |
|---|---|---|
| T2M | 66.8 | 60.8 |
| T3M | 65.1 | 56.4 |
| T6M | 59.2 | 48.7 |

| 40°C | | |
|---|---|---|
| T2M | 38.2 | 34.7 |
| T3M | 26.3 | 24.3 |

### Example 6: Hydrophobic interaction chromatography (HIC) for determination of succinimide content

### Method description

IgG1 samples were digested with CpB at a final concentration of 1 mg/mL in 20 mM MES, pH 6.5±0.05. The mobile phase "A" consisted of 1 M ammonium sulfate containing 0.035 M phosphate buffer, while mobile phase "B" was 0.045 M phosphate buffer containing 10%-15% propan-2-ol. The mobile phase pH was set to pH = 7, by adjusting the ratio of sodium dihydrogen phosphate and disodium hydrogen phosphate. Separations were performed on a MAbPac^{™} HIC-20 column operating at 0.7 mL/min flow rate. The gradient ran for 28 minutes (70%-20%-5%-70% mobile phase A and 30%-80%-95%-30% mobile phase B). Peak retention times were monitored at 214 nm.

### Results

Gradual decline in succinimide content observed for samples stored at 5°C and 25°C was most likely caused by hydrolysis of succinimide to Asp/isoAsp. Formulation F2 showed higher succinimide content at 5°C and 25°C compared to formulation F1 in the short-term stability study conducted in vials.

**Table 9: Hydrophobic interaction chromatography of formulations F1 and F2.**

| storage conditions | succinimide content [%] | |
|---|---|---|
| | formulation F1 | formulation F2 |
| T0 | 2.1 | 2.1 |

| 5°C | | |
|---|---|---|
| T1M | 1.5 | 1.7 |
| T2M | 1.4 | 1.7 |
| T3M | 1.3 | 1.5 |

| 25°C | | |
|---|---|---|
| T1M | 3.0 | 3.3 |
| T2M | 2.8 | 3.1 |
| T3M | 2.7 | 3.0 |

### Example 7: Light obscuration (LO) for determination of sub-visible particle content

### Method description

Light obscuration was performed by visual inspection of the sample using visual inspection station Apollo II of Adelphi Manufacturing Co. The sample was equilibrated to room temperature, swirled and particles were observed for 5 seconds in front of a white and a black panel. Observed particles were classified measured in four size channels: 2 µm, 5 µm, 10 µm and 25µm. Samples were analyzed in duplicate.

### Results

Relatively low numbers of particles were detected for all the formulations at each particle size channel. The data did not reveal an obvious trend (neither ascending or descending) for any formulation. Formulation F2 tended to contain a higher number of particles ≥ 10 µm during this study than formulation F1 in the short-term stability study conducted in vials.

**Table 10: Light obscuration of formulations F1 and F2.**

| storage conditions | particles ≥ 10 µm [#/ml] | |
|---|---|---|
| | formulation F1 | formulation F2 |
| T0 | 33 | 83 |

| 5°C | | |
|---|---|---|
| T1M | 17 | 33 |
| T2M | 0 | 50 |
| T3M | 17 | 17 |

| 25°C | | |
|---|---|---|
| T1M | 0 | 17 |
| T2M | 17 | 67 |
| T3M | 0 | 17 |

| 40°C | | |
|---|---|---|
| T2W | 17 | 0 |
| T1M | 0 | 0 |
| T2M | 17 | 0 |
| T3M | 50 | 67 |

Entyvio DP SC tended to contain a higher number of sub-visible particles in channels 2≤x<5 µm and 5≤x<10 µm than formulation F1 during the long-term stability study conducted in prefilled syringes.

**Table 11: Light obscuration of formulations F1 and Entyvio DP SC.**

| storage conditions | particles ≥ 10 µm [#/ml] | |
|---|---|---|
| | formulation F1 | Entyvio DP SC |
| T0 | 83 | 133 |

| 5°C | | |
|---|---|---|
| T6M | 33 | 33 |
| T12M | 33 | 150 |

| 25°C | | |
|---|---|---|
| T3M | 0 | 150 |
| T6M | 33 | 217 |

| 40°C | | |
|---|---|---|
| T3M | 0 | 183 |

**Table 12: Light obscuration of formulations F1 and Entyvio DP SC.**

| storage conditions | particles 5≤x<1 0 [#/ml] | |
|---|---|---|
| | formulation F1 | Entyvio DP SC |
| T0 | 650 | 4967 |

| 5°C | | |
|---|---|---|
| T6M | 183 | 2900 |
| T12M | 33 | 3300 |

| 25°C | | |
|---|---|---|
| T3M | 300 | 1717 |
| T6M | 817 | 1667 |

| 40°C | | |
|---|---|---|
| T3M | 33 | 3133 |

**Table 13: Light obscuration of formulations F1 and Entyvio DP SC.**

| storage conditions | particles 2≤x<5 µm [#/ml] | |
|---|---|---|
| | formulation F1 | Entyvio DP SC |
| T0 | 2200 | 12850 |

| 5°C | | |
|---|---|---|
| T6M | 517 | 13333 |
| T12M | 233 | 14750 |

| 25°C | | |
|---|---|---|
| T3M | 783 | 8150 |
| T6M | 2233 | 7633 |

| 40°C | | |
|---|---|---|
| T3M | 350 | 17783 |

### Example 7: Capillary electrophoresis with sodium dodecyl sulfate (CE-SDS) for determination of purity

### Method description

Capillary electrophoresis of sodium dodecyl sulfate (CE-SDS) under reducing conditions is a technique used to determine the purity of proteins. The sample matrix is replaced with water prior to analysis. The sample is prepared in sample buffer (100 mM Tris-HCl, pH 9.0, 1% SDS) and reduced with 2-mercaptoethanol. The final concentration of the sample is 1.2 mg/ml. Separation of protein fragments is performed on a bare fused silica capillary (30.2 cm, 50 µm ID) filled with SDS-MW gel buffer at a voltage of 15 kV. Protein fragments are detected under 214 nm UV light.

### Results

The results of CE-SDS in reduced conditions analysis did not show marked changes in purity over the storage time for samples stored at 5°C. The samples stored at 25°C and 40°C showed gradual decrease in purity. Entyvio DP SC presented lower purity compared to formulation F1 in the long-term stability study conducted in prefilled syringes. The downward trend in purity is higher for Entyvio DP SC under accelerated and stressed conditions.

**Table 14: Capillary electrophoresis-sodium dodecyl sulphate in reduced conditions of formulations F1 and Entyvio DP SC.**

| storage conditions | purity [%] | |
|---|---|---|
| | formulation F1 | Entyvio DP SC |
| T0 | 98.7 | 98.2 |

| 5°C | | |
|---|---|---|
| T6M | 99.0 | 98.0 |
| T12M | 98.3 | 97.8 |

| 25°C | | |
|---|---|---|
| T2M | 98.0 | 96.7 |
| T3M | 97.2 | 95.9 |
| T6M | 97.7 | 95.3 |

| 40°C | | |
|---|---|---|
| T2M | 95.0 | 92.9 |
| T3M | 92.9 | 89.6 |

### SEQUENCE LISTING

SEQ ID NO: 1 - vedolizumab heavy chain 
QVQLVQSGAE VKKPGASVKV SCKGSGYTFT SYWMHWVRQA PGQRLEWIGE 50
IDPSESNTNY NQKFKGRVTL TVDISASTAY MELSSLRSED TAVYYCARGG 100
YDGWDYAIDY WGQGTLVTVS SASTKGPSVF PLAPSSKSTS GGTAALGCLV 150
KDYFPEPVTV SWNSGALTSG VHTFPAVLQS SGLYSLSSVV TVPSSSLGTQ 200
TYICNVNHKP SNTKVDKKVE PKSCDKTHTC PPCPAPELAG APSVFLFPPK 250
PKDTLMISRT PEVTCVVVDV SHEDPEVKFN WYVDGVEVHN AKTKPREEQY 300
NSTYRVVSVL TVLHQDWLNG KEYKCKVSNK ALPAPIEKTI SKAKGQPREP 350
QVYTLPPSRD ELTKNQVSLT CLVKGFYPSD IAVEWESNGQ PENNYKTTPP 400
VLDSDGSFFL YSKLTVDKSR WQQGNVFSCS VMHEALHNHY TQKSLSLSPG 450
K 451
SEQ ID NO: 2 - vedolizumab light chain

DVVMTQSPLS LPVTPGEPAS ISCRSSQSLA KSYGNTYLSW YLQKPGQSPQ 50
LLIYGISNRF SGVPDRFSGS GSGTDFTLKI SRVEAEDVGV YYCLQGTHQP 100
YTFGQGTKVE IKRTVAAPSV FIFPPSDEQL KSGTASVVCL LNNFYPREAK 150
VQWKVDNALQ SGNSQESVTE QDSKDSTYSL SSTLTLSKAD YEKHKVYACE 200
VTHQGLSSPV TKSFNRGEC 219

The following list of items describes preferred embodiments.

### EMBODIMENTS

1. A pharmaceutical formulation comprising Vedolizumab, histidine, arginine, and sorbitol.
2. The pharmaceutical formulation according to item 1, wherein the concentration of sorbitol is between 50 mM and 200 mM.
3. The pharmaceutical formulation according to item 1 or 2, wherein the concentration of sorbitol is about 100 mM.
4. The pharmaceutical formulation according to any one of items 1 to 3, wherein the concentration of Vedolizumab is between 150 mg/ml and 170 mg/ml, in particular about 158.8 mg/ml.
5. The pharmaceutical formulation according to any one of items 1 to 4, wherein the concentration of arginine is between 150 mM and 200 mM, in particular about 180 mM.
6. The pharmaceutical formulation according to any one of items 1 to 5, wherein arginine is arginine HCl.
7. The pharmaceutical formulation according to any one of items 1 to 6, wherein the concentration of histidine is between 20 mM and 100 mM, in particular about 50 mM.
8. The pharmaceutical formulation according to any one of items 1 to 7, wherein histidine is a mixture of L-histidine and L-histidine HCl.
9. The pharmaceutical formulation according to any one of items 1 to 8, wherein the pH of the pharmaceutical formulation is between 6 and 7, in particular about 6.5.
10. The pharmaceutical formulation according to any one of items 1 to 9, further comprising a surfactant.
11. The pharmaceutical formulation according to item 10, wherein the surfactant is poloxamer 188.
12. The pharmaceutical formulation according to item 11, wherein the concentration of poloxamer 188 is between 0.02% (w/v) and 0.1% (w/v), in particular 0.06% (w/v).
13. The pharmaceutical formulation according to any one of items 1 to 12, wherein the pharmaceutical formulation does not comprise citrate, and in particular does not comprise any antioxidant or chelator.
14. The pharmaceutical formulation according to any one of items 1 to 13, being a liquid pharmaceutical composition, especially an aqueous pharmaceutical formulation.
15. The pharmaceutical formulation according to any one of items 1 to 14, consisting of Vedolizumab, sorbitol, arginine, histidine, a surfactant and water.
16. The pharmaceutical formulation according to any one of items 1 to 15, consisting of water, about 158.8 mg/ml Vedolizumab, about 100 mM sorbitol, about 180 mM arginine, about 50 mM histidine, and about 0.06% (w/v) poloxamer 188, and has a pH of about 6.5, wherein "about" refers to a variation by +/- 5% of the indicated value.
17. A sealed container containing the pharmaceutical formulation according to any one of items 1 to 16.
18. An article of manufacture comprising the container according to item 17.
19. The pharmaceutical formulation according to any one of items 1 to 16 or the article of manufacture according to item 18 for use in the treatment of inflammatory bowel disease such as ulcerative colitis, Crohn's disease and pouchitis, or of graft versus host disease.
20. The pharmaceutical formulation according to any one of items 1 to 16 or the article of manufacture according to item 18 for use according to item 19, wherein the treatment comprises subcutaneous administration of the pharmaceutical formulation to a patient in need thereof.

## Claims

1. A pharmaceutical formulation comprising Vedolizumab, histidine, arginine, and sorbitol.

2. The pharmaceutical formulation according to claim 1, wherein the concentration of sorbitol is between 50 mM and 200 mM.

3. The pharmaceutical formulation according to any one of claims 1 to 2, wherein the concentration of Vedolizumab is between 150 mg/ml and 170 mg/ml.

4. The pharmaceutical formulation according to any one of claims 1 to 3, wherein the concentration of arginine is between 150 mM and 200 mM.

5. The pharmaceutical formulation according to any one of claims 1 to 4, wherein arginine is in the form of the free base.

6. The pharmaceutical formulation according to any one of claims 1 to 5, wherein histidine is in the form of the free base, wherein optionally the concentration of histidine is between 20 mM and 100 mM.

7. The pharmaceutical formulation according to any one of claims 1 to 6, further comprising a surfactant.

8. The pharmaceutical formulation according to claim 7, wherein the surfactant is poloxamer 407.

9. The pharmaceutical formulation according to any one of claims 1 to 8, being a liquid pharmaceutical composition, especially an aqueous pharmaceutical formulation.

10. A sealed container containing the pharmaceutical formulation according to any one of claims 1 to 9.

11. An article of manufacture comprising the container according to claim 10.

12. The pharmaceutical formulation according to any one of claims 1 to 9 or the article of manufacture according to claim 11 for use in the treatment of inflammatory bowel disease such as ulcerative colitis, Crohn's disease and pouchitis, or of graft versus host disease.
